# EUROPEAN PATENT APPLICATION

(11) **EP 2 425 861 A1**
(43) Date of publication of application: **07.03.2012**
(21) Application number: 09843991.2
(22) Date of filing: 28.04.2009
(51) Int. Cl.: A61K 51/00, A61P 35/00

(54) **RADIOACTIVELY LABELED SUBSTANCE**

(71) Applicant: National University Corporation Chiba University, Chiba-shi Chiba 263-8522 (JP)
(72) Inventor: ARANO, Yasushi, Chiba-shi Chiba 260-8670 (JP); UEHARA, Tomoya, Chiba-shi Chiba 260-8670 (JP)
(74) Representative: De Clercq, Ann G. Y.
(86) International application number: PCT/JP2009/058372
(87) International publication number: WO 2010/125647

(57) **Abstract**

Provided are: a radiolabeled drug, which is efficiently accumulated in a target and has high *in vivo* stability; and diagnosis and treatment each using the radiolabeled drug. Specifically provided are: a radiolabeled drug showing increased accumulation in a target site, which comprises a complex composed of a ligand that is bound to a compound capable of binding to a target molecule and forms a polycoordinated complex with a metal (e.g., technetium or rhenium) and a radionuclide of the metal; the radiolabeled drug for diagnosis or treatment; a ligand for preparing the radiolabeled drug; a kit that comprises a drug comprising the ligand and a drug comprising a radionuclide of a metal, as separate package units; and a method of increasing accumulation of a radiolabeled drug in a target site, comprising using the above-mentioned radiolabeled drug.

## Description

### Technical Field

The present invention relates to a radiolabeled drug, which is highly accumulated in a target site. Specifically, the present invention relates to a radiolabeled drug showing increased accumulation in a target site, which comprises a complex composed of a ligand that is bound to a compound capable of binding to a target molecule and forms a polycoordinated complex with a metal, and a radionuclide of the metal. More specifically, the present invention relates to a radiolabeled drug for diagnosis or treatment, which comprises a complex composed of a ligand that is bound to a compound capable of binding to a target molecule and forms a polycoordinated complex with technetium (Tc) or rhenium (Re), and any one of radionuclides of metals selected from the group consisting of ^{99m}technetium, ¹⁸⁶rhenium, and ¹⁸⁸rhenium. The present invention also relates to a ligand for preparing the radiolabeled drug. The present invention also relates to a kit that comprises a drug comprising the ligand and a drug comprising a radionuclide of a metal, as separate package units. The present invention also relates to a method of increasing accumulation of a radiolabeled drug in a target site, comprising using the above-mentioned radiolabeled drug.

### Background Art

A radiolabeled drug is a drug comprising a compound labeled with a nuclide of a radioisotope, and is widely utilized in, for example, diagnosis and treatment of diseases, e.g., diagnosis and treatment of tumors. The accumulation of the radiolabeled drug in a certain tissue or cell can provide highly sensitive diagnosis and effective treatment and reduce side effects on normal tissues and cells. For example, even when tumor cells metastasize and spread to organs and tissues, effective diagnosis and treatment can be performed without affecting normal tissues and cells. In diagnosis and treatment using the radiolabeled drug, the selection of a useful nuclide and drug design for accumulating the drug in a certain tissue and cell have been conducted.

Technetium-99m (^{99m}Tc) has a half-life (6 hours) suitable for diagnostic imaging, and emits only γ-rays with energy (140 KeV) suitable for external counting of radiation. Further, Technetium-99m is currently the most commonly used radionuclide (RI) for nuclear medicine diagnostic imaging because it is easily available from a generator system using radiation equilibrium between Technetium-99m and molybdenum-99 (⁹⁹Mo) (⁹⁹Mo/^{99m}Tc generator).

A ^{99m}Tc preparation is primarily synthesized by a complex forming reaction of ^{99m}Tc with a compound in which a ligand capable of forming a stable complex with ^{99m}Tc is bound to a target molecule recognition element such as an anti-tumor antibody and a low molecular peptide (referred to as ligand derivative). A complex that is composed of a tetradentate ligand N₂S₂ and pentavalent Tc at a molar ratio of 1:1 is known as such ^{99m}Tc preparation, for example.

In the production of the ^{99m}Tc preparation, the ligand derivative is used at a high concentration in order to obtain a trace amount of the ^{99m}Tc preparation in a high radiochemical yield in a short period of time. As a result, in a ^{99m}Tc-labeled solution, a large excess amount of the ligand derivative coexists with a trace amount of a ^{99m}Tc-labeled drug having a target molecule recognition element and competes with the ^{99m}Tc-labeled drug in regard to binding to a target molecule, and thus, the accumulation of the ^{99m}Tc-labeled drug in a target site is greatly impaired.

However, it is not easy to aseptically remove the ligand derivative that has not formed a complex before the administration. This may impair such a property of ^{99m}Tc that allows a simple operation. A ligand that can be used even at a low concentration to provide the ^{99m}Tc complex in a high yield is also investigated. However, it is considered that the production of such a ligand is difficult to be accomplished because the concentration of ^{99m}Tc is extremely low.

Technetium forms a six-coordinated, three-coordinated, or two-coordinated complex that is stable *in vivo* with a suitable monodentate, bidentate, or tridentate ligand (Non Patent Literatures 1 and 2). In actuality, ligands, which give complexes of technetium with the ligands at 1:2, 1:3, and 1:6, have also been reported (Non Patent Literatures 1, 2, and 4). However, it is not obvious whether those ligands produce stable complexes in vino with Tc even when the recognition element is introduced.

Rhenium is a transition element belonging to Group VII like technetium, and has chemical nature similar to technetium. ¹⁸⁶Rhenium (¹⁸⁶Re) and ¹⁸⁸Rhenium (¹⁸⁸Re) are known as the radioisotopes of rhenium. ¹⁸⁶Re and ¹⁸⁸Re both emit β-rays with high energy, and have half-lives of 90.6 hours and 16.9 hours, respectively. The β-rays with high energy exhibits cytotoxicity, and hence a ¹⁸⁶Re-labeled drug has been studied as a therapeutic drug for cancer diseases. For example, ¹⁸⁶ Re-labeled hydroxyethylidene diphosphonate (¹⁸⁶Re-HEDP) has been reported to have a pain relief effect on metastatic bone tumor. However, ¹⁸⁶Re-HEDP involves problems of delayed blood clearance and high accumulation in stomach because of its low *in vivo* stability.

As described above, the development of a radiolabeled drug exhibiting high accumulation in a certain tissue or cell and having high in vino stability is required in the field of diagnosis and treatment of diseases.

### Citation List

### Non Patent Literature

[NPL 1] Wust F, Carlson KE, Katzenellenbogen JA, Spies H, and Johannsen B. Synthesis and binding affinities of new 17 alpha-substituted estradiol-rhenium "n+1" mixed-ligand and thioether-carbonyl complexes. Steroids 1998: 63: 665-71
[NPL 2] Jurisson SS, and Lydon JD. Potential technetium small molecule radiopharmaceuticals. Chem Rev 1999: 99: 2205-18 [NPL 3] Mulder A, Huskens J, and Reinhoudt DN. Multivalency in supramolecular chemistry and nanofabrication. Org Biomol Chem 2004: 2: 3409-24
[NPL 4] Arano Y. Recent advances in 99mTc radiopharmaceuticals. J Nucl Radiochem Sci 2005: 6: 177-181

### Summary of Invention

### Problem to be Solved by the Invention

The present invention aims at solving the above-mentioned problems of prior art techniques. That is, an object of the present invention is to provide a radiolabeled drug, which is efficiently accumulated in a target and has high in vino stability, and to provide diagnosis and treatment using the radiolabeled drug.

### Means for Solving the Problem

The inventors of the present invention have considered as follows: a complex composed of a ligand that has a recognition element having one binding site to a target molecule (monovalent recognition element) and forms a polycoordinated complex with a metal, and the complex acquires a strong avidity to the target molecule because the complex is a polyvalent complex having the same number of recognition elements as that of ligands; and as a result, after a complex forming reaction, the complex can be highly accumulated in a peripheral target molecule even when the complex is administered in a state in which a ligand that has not formed a complex is included therein. Thus, the inventors have made extensive studies to accomplish the above-mentioned object. D-penicillamine (sometimes abbreviated as D-Pen), which gives a complex of pentavalent technetium (Tc) with a ligand at 1:2 (two-coordinated complex) and 1-amino-2-methylpropane-2-thiol-N-acetate (hereinafter, sometimes abbreviated as AMPT), which is different from D-Pen in configuration of a coordinating group were used as the ligands, and a cyclic pentapeptide c(RGDfK) having an affinity to integrin highly expressed in neovascular vessels in cancer was used as the target molecule recognition element, to synthesize a ligand to which c(RGDfK) is bound. Then, the inventors have prepared a ^{99m}Tc complex having two target molecule recognition elements (bivalent complex) by subjecting the ligand to a reaction with ^{99m}Tc. In addition, from studies on the pharmacokinetics of the ^{99m}Tc complex, the inventors have found that the complex is sufficiently stable *in vivo* as well. The ^{99m}Tc complex has two target molecule recognition elements, and hence has a stronger avidity to a target molecule and is highly accumulated in a peripheral target molecule as compared to a ^{99m}Tc complex having one target molecule recognition element. Further, the inventors have found that use of rhenium (Re) in place of ^{99m}Tc described above can allow formation of a complex similar to the complex described above. The present invention has been accomplished based on those findings.

That is, the present invention relates to the following items.
(1) A radiolabeled drug showing increased accumulation in a target site, comprising a complex composed of a ligand that is bound to a compound capable of binding to a target molecule and forms a polycoordinated complex with a metal, and a radionuclide of the metal.
(2) A ^{99m}Tc-labeled drug for diagnosis showing increased accumulation in a target site, comprising a complex composed of D-penicillamine or 1-amino-2-methylpropane-2-thiol-N-acetate, which is a ligand that is bound to a compound capable of binding to a target molecule and forms a two-coordinated or three-coordinated complex with pentavalent technetium (Tc), and ^{99m}Tc_{.}
(3) A ^{99m}Tc-labeled drug for diagnosis according to the above-mentioned items, wherein the ligand that is bound to a compound capable of binding to a target molecule is a ligand that is bound to a cyclic pentapeptide c(RGDfK).
(4) A ¹⁸⁶Re- or ¹⁸⁸Re-labeled drug for treatment showing increased accumulation in a target site, comprising a complex composed of D-penicillamine or 1-amino-2-methylpropane-2-thiol-N-acetate, which is a ligand that is bound to a compound capable of binding to a target molecule and forms a two-coordinated or three-coordinated complex with pentavalent rhenium (Re), and ¹⁸⁶Re or ¹⁸⁸Re_{.}
(5) A ¹⁸⁶Re- or ¹⁸⁸Re-labeled drug for treatment according to the above-mentioned items, wherein the ligand that is bound to a compound capable of binding to a target molecule is a ligand that is bound to a cyclic pentapeptide c(RGDfK).
(6) A radiolabeled drug according to any one of the above-mentioned items, wherein the radiolabeled drug is used in diagnosis or treatment.
(7) A ligand for preparing a radiolabeled drug for diagnosis or treatment showing increased accumulation in a target site, the ligand being bound to a compound capable of binding to a target molecule and forming a polycoordinated complex with a metal.
(8) A ligand for preparing a radiolabeled drug according to the above-mentioned items, wherein a radionuclide of the metal includes any one of radionuclides of metals selected from the group consisting of ^{99m}technetium, ¹⁸⁶rhenium, and ¹⁸⁸rhenium.
(9) A ligand for preparing a radiolabeled drug according to the above-mentioned items, wherein the ligand that forms a polycoordinated complex with a metal is D-penicillamine or 1-amino-2-methylpropane-2-thiol-N-acetate, which forms a two-coordinated or three-coordinated complex with pentavalent technetium or rhenium.
(10) A ligand for preparing a radiolabeled drug according to the above-mentioned items, whrerein the ligand that is bound to a compound capable of binding to a target molecule is a ligand that is bound to a cyclic pentapeptide c(RGDfK).
(11) A kit, comprising, as separate package units, a drug that comprises the ligand for preparing a radiolabeled drug of any one of the above-mentioned items, and a drug that comprises a radionuclide of a metal that forms a polycoordinated complex with the ligand.
(12) A method of producing a compound represented by the following formula (I): 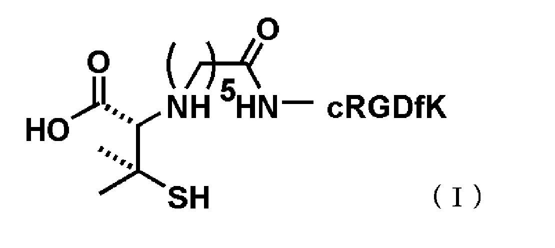 the method comprising using a compound represented by the following formula (II): 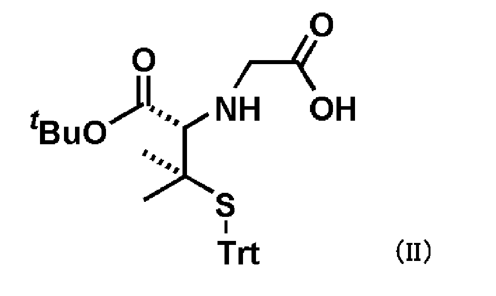
(13) A method of increasing accumulation of a radiolabeled drug in a target site, the method comprising using a radiolabeled drug that comprises a complex composed of a ligand that is bound to a compound capable of binding to a target molecule and forms a polycoordinated complex with a metal, and a radionuclide of the metal.
(14) A method according to the above-mentioned items, wherein the radionuclide of the metal is any one of radionuclides of metals selected from the group consisting of ^{99m}technetium, ¹⁸⁶rhenium, and ¹⁸⁸rhenium.
(15) A method according to the above-mentioned items, wherein the ligand that forms a polycoordinated complex with a metal is D-penicillamine or 1-amino-2-methylpropane-2-thiol-N-acetate, which forms a two-coordinated or three-coordinated complex with pentavalent technetium or rhenium.
(16) A method according to the above-mentioned items, wherein the ligand that is bound to a compound capable of binding to a target molecule is a ligand that is bound to a cyclic pentapeptide c(RGDfK).

### Advantageous Effect of the Invention

According to the present invention, the radiolabeled drug, which is efficiently accumulated in a target site and has sufficient *in vivo* stability, can be provided. The radiolabeled drug according to the present invention comprises a complex composed of a ligand that has a target molecule recognition element having one binding site to a target molecule (monovalent target molecule recognition element) and forms a polycoordinated complex with a metal, and a radionuclide of the metal. The complex is a polyvalent complex having the same number of target molecule recognition elements as that of ligands for forming the complex and is hence highly accumulated in a target site as compared to a conventionally used monovalent complex. Thus, the use of the radiolabeled drug according to the present invention can increase the accumulation of the radiolabeled drug in a target site as compared to those conventionally used. As a result, even when the radiolabeled drug contains a ligand that has not formed a complex, high sensitivity and effects can be provided in diagnostic imaging and internal radiation therapy for cancer diseases and the like each using the radiolabeled drug. Specifically, for example, a ^{99m}Tc-labeled drug, a ¹⁸⁶Re-labeled drug, and a ¹⁸⁸Re-labeled drug, each of which is efficiently accumulated in a target site and has sufficient *in vivo* stability, can be provided. The ^{99m}Tc-labeled drug according to the present invention is useful as a diagnostic drug for diagnostic imaging using a radiolabeled drug, and the ¹⁸⁶Re-labeled drug and the ¹⁸⁸Re-labeled drug are useful for internal radiation therapy for cancers.

### Brief Description of Drawings

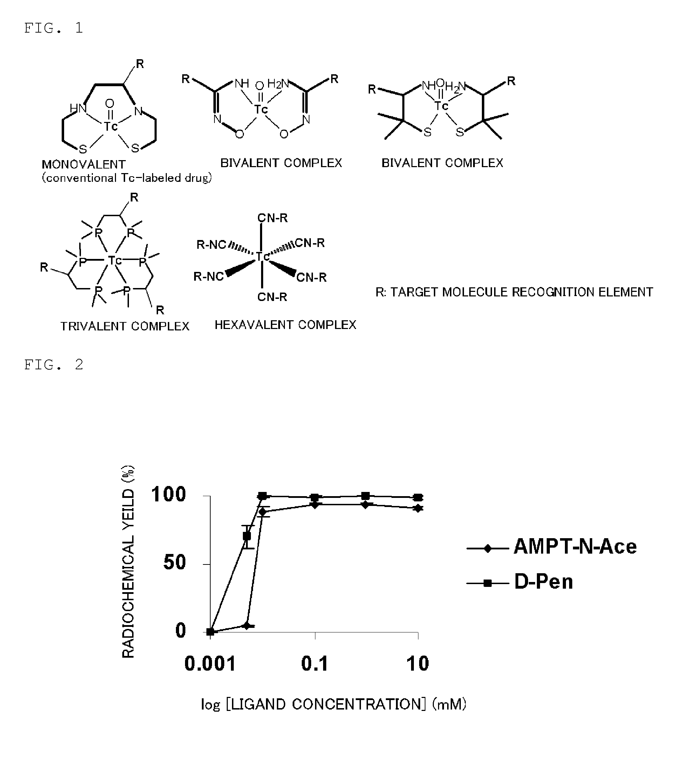

FIG. 1 is a schematic diagram illustrating structures of polyvalent ^{99m}Tc-labeled drugs.
FIG. 2 is a graph showing an effect of a ligand concentration on formation of a technetium complex. In the figure, solid squares and solid diamonds denote cases of using as ligands D-Pen and AMPT-N-acetate (referred to as AMPT-N-Ace), respectively.

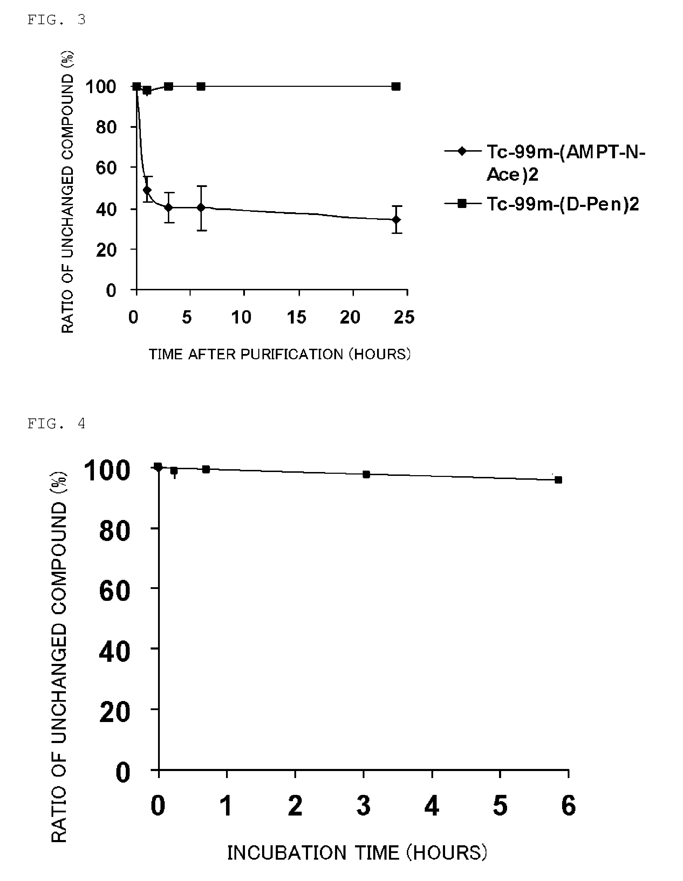

FIG. 3 is a graph showing the stability of ^{99m}Tc- (D-Pen) and ^{99m}Tc- (AMPT-N-acetate)₂ in a buffer. In the figure, solid squares and solid diamonds denote ^{99m}Tc- (D-Pen)₂ (referred to as Tc-99m- (D-Pen)₂) and ^{99m}Tc-(AMPT-N-acetate)₂ (referred to Tc-99m-(AMPT-N-acetate)₂), respectively.
FIG. 4 is a graph showing the stability of 99^{m}Tc- (D-Pen-Hx-cRGDfK)₂ in a buffer.

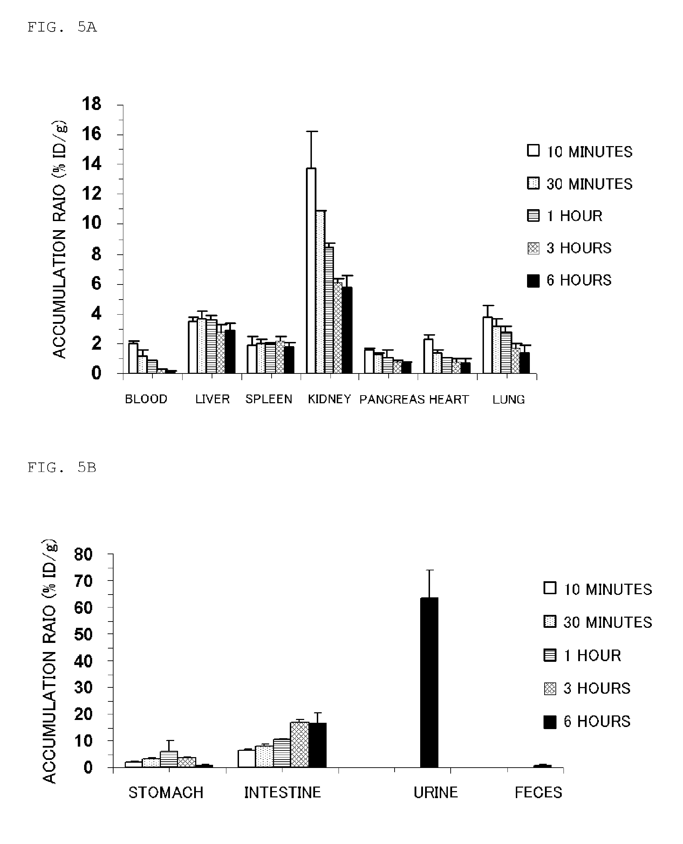

FIG. 5A is a graph showing pharmacokinetics of purified ^{99m}Tc-(D-Pen-Hx-cRGDfK)₂ administered to mice using accumulation ratios in different organs and samples. The accumulation ratios are each represented by a value obtained by dividing a ratio (%) of a radioactivity in each organ to an administered radioactivity by a weight of each organ.
FIG. 5B is a graph showing pharmacokinetics of purified ^{99m}Tc-(D-Pen-Hx-cRGDfK)₂ administered to mice using accumulation ratios in different organs and samples. The accumulation ratios are each represented by a value obtained by dividing a ratio (%) of a radioactivity in each organ or each sample to an administered radioactivity by a weight of each organ.

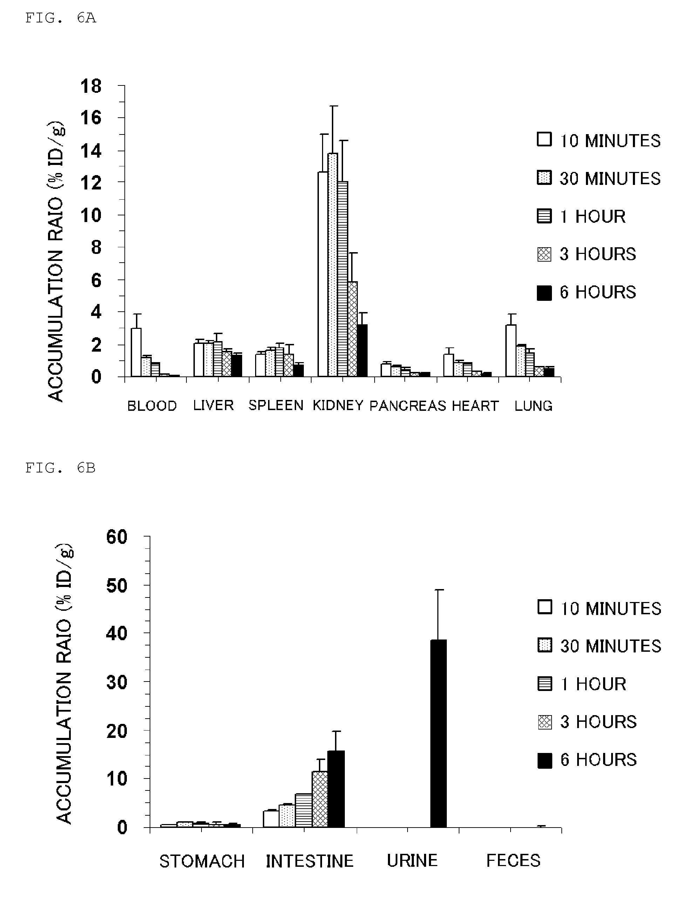

FIG. 6A is a graph showing pharmacokinetics of unpurified ^{99m}Tc-(D-Pen-Hx-cRGDfK)₂ administered to mice using accumulation ratios in different organs and samples. The accumulation ratios are each represented by a value obtained by dividing a ratio (%) of a radioactivity in each organ to an administered radioactivity by a weight of each organ.
FIG. 6B is a graph showing pharmacokinetics of unpurified ^{99m}Tc-(D-Pen-Hx-cRGDfK)₂ administered to mice using accumulation ratios in different organs and samples. The accumulation ratios are each represented by a value obtained by dividing a ratio (%) of a radioactivity in each organ or each sample to an administered radioactivity by a weight of each organ.

### Description of Embodiments

The present invention relates to a radiolabeled drug showing increased accumulation in a target site, which comprises a complex composed of a ligand that is bound to a target molecule recognition element and forms a polycoordinated complex with a metal, and a radionuclide of the metal.

The term "complex" means a substance in which a ligand is coordinated to an atom or an ion of a metal and a metal-like element at the center. The coordination means that a ligand forms a coordination bond with a center metal and is arranged around the center metal. The complex is formed by the coordination bond of the ligand and the metal. The coordination bond refers to a bond in which two atomic valence electrons involved in one bond are supplied from one atom alone. Polycoordination means that a plurality of ligands form coordination bonds with a center metal and are arranged around the center metal. n-Coordination means that n ligand molecules form coordination bonds with a center metal and are arranged around the center metal (when the metal is a transition metal, n generally represents 2 to 9). That is, the polycoordinated complex refers to a complex composed of a plurality of ligand molecules that form coordination bonds with a center metal. In the complex, the number of the ligands that are arranged around the center metal and form coordination bonds is referred to as coordination number.

The term "ligand" means a compound containing another atom (coordinating atom) that forms a coordination bond with a center metal in a complex. There are many types of ligands, a compound containing two or more potential coordinating atoms is referred to as polydentate ligand, and compounds containing one, two, and three coordinating atoms are referred to as monodentate ligand, bidentate ligand, and tridentate ligand, respectively.

The term "target molecule recognition element" means a compound capable of binding to a target molecule, preferably a compound capable of specifically binding to a target molecule. The binding specifically to a target molecule means that a compound is capable of binding to a target molecule but does not bind to or weakly binds to a molecule other than the target molecule. Examples of the target molecule recognition element include a protein, a peptide, an antibody, and an antibody fragment. The binding of the target molecule recognition element to the ligand to form a complex with the metal allows the binding of the complex to a target molecule, i.e., a target site. Therefore, the complex can be made to accumulate in a desired target site by selecting and using the target molecule recognition element.

The metal used in the present invention that forms the complex is a metal that belongs to a transition metal and forms two or more coordination bonds with the ligand. A charge of a metal atom is not particularly limited, and there are given, for example, metal atoms having monovalent, trivalent, or pentavalent charges. A radionuclide of the metal is preferably used as the metal. Preferred specific examples of the radionuclide of the metal include ^{99m}Tc, ¹⁸⁶Re, and¹⁸⁸Re. The radionuclide of the metal is not limitedto those specific examples, and any radionuclide of the metal can be used as long as the radionuclide has a radiation, a radiation dose, and a half-life suitable for the purposes such as diagnosis using the radiolabeled drug and internal radiation therapy for the cancer disease and the like. A radionuclide of a metal having a short half-life is preferably used from the viewpoint of reducing an effect on normal tissues and cells in diagnosis and treatment.

In the present invention, a compound capable of forming a polycoordinated complex by a coordination bond with a metal belonging to a transition metal is used as the ligand that forms the complex. For example, as a compound capable of forming a polycoordinated complex by a coordination bond with ^{99m}Tc, there are given, D-penicillamine (sometimes abbreviated as D-Pen), 1-amino-2-methylpropane-2-thiol-N-acetate (hereinafter, sometimes abbreviated as AMPT), and hydroxamamide, each of which gives a complex composed of pentavalent Tc and the ligand at 1:2 (those ligands each form a bivalent complex), thiourea, dimethylphosphinoethane, and o-phenylenebis (dimethylarsine), each of which gives a complex composed of trivalent Tc and the ligand at 1: 3 (those ligands each form a trivalent complex), and isonitrile, which gives a complex composed of monovalent Tc and the ligand at 1:6 (this ligand forms a hexavalent complex). Meanwhile, rhenium is a transition metal belonging to Group VII like technetium and has chemical nature similar to technetium. Thus, the ligand that forms a complex with technetium also forms a similar complex with rhenium. Therefore, any of the ligands exemplified above can be used as a ligand that forms complexes with ¹⁸⁶Re and ¹⁸⁸Re. The ligands are not limited to those specific examples, and any compound can be used as long as the compound forms a polycoordinated complex with a metal.

The ligand can contain a crosslinking compound, which allows the binding of the ligand to the target molecule recognition element, in its molecule. Any compound can be used as the crosslinking compound as long as the compound can crosslink the target molecule recognition element to the ligand and does not inhibit the binding of the target molecule recognition element to the target molecule. Preferred examples thereof include hexanoic acid and polyethylene glycol. A molecular weight of polyethylene glycol is preferably 200 or more, more preferably 400 or more, still more preferably 600 or more and suitably 1,500 or less.

In the present invention, examples of the target molecule recognition element include a protein, a peptide, an antibody, and an antibody fragment. Specific examples of the target molecule recognition element include a ligand, an antibody, and an Fab fragment of an antibody, each of which is capable of binding to a protein highly expressed in tissue construction associated with inflammation and tumor cell infiltration or a protein specifically expressed in tumor cells. More specific examples of the target molecule recognition element include a cyclic pentapeptide c(RGDfK) having an affinity to integrin that is highly expressed in neovascular vessels in cancers. Further examples of the target molecule recognition element include bis-phosphonic acid, oligo-aspartic acid, and oligo-glutamic acid, each of which has an affinity to hydroxyapatite present abundantly in osteogenic cancer (bone metastasis), a peptide (fMet-Leu-Phe), which has an affinity to a receptor of a chemotactic factor that is present on the surface of macrophages, and folic acid and its derivatives, each of which is capable binding to a folic acid receptor that is expressed on cancer cells. The target molecule recognition element is not limited to the exemplified compounds, and any compound can be used as long as the compound is capable of binding to a target molecule.

The radiolabeled drug according to the present invention comprises as an active ingredient a complex composed of a ligand that is bound to a target molecule recognition element and forms a polycoordinated complex with a metal, and a radionuclide of the metal. Such complex is composed of the ligand that is bound to a target molecule recognition element and forms a polycoordinated complex with a metal, and thus has the same number of target molecule recognition elements as that of ligands (see FIG. 1). That is, such complex has a plurality of binding sites to a target molecule. As described above, the complex composed of a ligand that is bound to a target molecule recognition element and forms a polycoordinated complex with a metal and consequently having the same number of target molecule recognition elements as that of ligands in the complex is referred to as polyvalent complex. A radiolabeled drug comprising the polyvalent complex as an active ingredient is referred to as polyvalent radiolabeled drug. For example, a complex composed of a ligand that is bound to a target molecule recognition element and forms a two-coordinated complex with a pentavalent metal has two binding sites to a target molecule in the complex and is referred to as bivalent complex. Further, a complex composed of a ligand that is bound to a target molecule recognition element and forms a three-coordinated complex with a trivalent metal has three binding sites to a target molecule in the complex and is referred to as trivalent complex. A complex composed of a ligand that is bound to a target molecule recognition element and forms a six-coordinated complex with a monovalent metal has six binding sites to a target molecule in the complex and is referred to as hexavalent complex.

It is known that a compound having two binding sites to a target molecule (bivalent compound) exhibits a high affinity to the target molecule and high accumulation in the target molecule as compared to a compound having one binding site to a target molecule (monovalent compound) (Non Patent Literature 3). When taking an antibody as an example of a compound capable of binding to a target molecule, a bivalent IgG antibody has at least 50 to 100 times higher avidity to an antigen than a monovalent Fab fragment, and a polyvalent IgM antibody has 104 times higher avidity to an antigen than the bivalent IgG antibody.

Thus, the polyvalent complex exhibits a high affinity to a target molecule and high accumulation in the target molecule as compared to the monovalent complex. Therefore, the radiolabeled drug comprising the polyvalent complex exhibits high accumulation in a target site.

The radiolabeled drug according to the present invention can be used for diagnostic imaging and internal radiation therapy using the radiolabeled drug. The radiolabeled drug according to the present invention is preferably used for diagnosis and treatment of cancer diseases. However, a disease to which the radiolabeled drug is applicable is not limited to the above-mentioned diseases. The radiolabeled drug can be applied to any disease to which diagnostic imaging or the internal radiation therapy is applicable. The target molecule recognition element to be bound to the complex that is the active ingredient of the radiolabeled drug is selected according to characteristics of a target subjected to diagnosis and treatment, which enables to diagnose and treat the target and allows the radiolabeled drug widely applicable in the field of diagnosis and treatment.

An administration route of the radiolabeled drug according to the present invention can preferably include intravenous administration and intra-arterial administration. The administration route is not limited to those routes, and any route can be used as long as the action of the radiolabeled drug can be effectively exerted after the administration of the radiolabeled drug.

An radioactive intensity of the radiolabeled drug according to the present invention is any intensity as long as the purpose can be accomplished by administering the radiolabeled drug and a clinical dosage is set so as to make radiation exposure to a subj ect as low as possible. The radioactive intensity canbe determined with reference to the radioactive intensity used in general diagnostic and therapeutic methods using a radiolabeled drug.

The radiolabeled drug according to the present invention not only can contain the above-mentioned complex as an active ingredient but also can contain one kind or two or more kinds of pharmaceutically acceptable carriers (pharmaceutical carriers), as necessary. Examples of the pharmaceutical carriers include an acid and a base for adjusting a pH, a buffer, a stabilizer, a tonicity agent, and a preservative.

The radiolabeled drug according to the present invention is preferably provided as a kit that comprises, as separate package units, a drug comprising a ligand that is bound to a target molecule recognition element and forms a polycoordinated complex with a metal, and a drug comprising a radionuclide of the metal.

One aspect of the radiolabeled drug according to the present invention can be a ^{99m}Tc-labeled drug. The ^{99m}Tc-labeled drug according to the present invention comprises a ^{99m}Tc complex composed of a ligand that is bound to a target molecule recognition element and forms a polycoordinated complex with technetium, and ^{99m}Tc. The ^{99m}Tc-labeled drug according to the present invention can be preferably used as a diagnostic drug for diagnostic imaging using a radiolabeled drug because ^{99m}Tc emits only γ rays with energy suitable for external counting of radiation.

The ligand that forms the ^{99m}Tc complex that is the active ingredient of the ^{99m}Tc-labeled drug according to the present invention is a ligand that is bound to a target molecule recognition element and forms a polycoordinated complex with technetium, and is used for preparing the ^{99m}Tc-labeled drug according to the present invention. Any ligand can also be used in the present invention as long as the ligand is a compound capable of forming a polycoordinated complex with ^{99m}Tc by a coordination bond. As the ligand for ^{99m}Tc, there are given, for example, D-Pen, AMPT, and hydroxamamide, each of which gives a complex composed of pentavalent Tc and the ligand at 1: 2 (those ligands each form a bivalent complex), thiourea, dimethylphosphinoethane, and o-phenylenebis(dimethylarsine), each of which gives a complex composed of trivalent Tc and the ligand at 1:3 (those ligands each form a trivalent complex), and isonitrile, which gives a complex composed of monovalent Tc and the ligand at 1:6 (this ligand forms a hexavalent complex).

The ^{99m}Tc complex that is the active ingredient of the ^{99m}Tc-labeled drug according to the present invention is a complex composed of a ligand that is bound to a target molecule recognition element and forms a polycoordinated complex with Tc, and ^{99m}Tc. The ligand that forms a polycoordinated complex means a ligand that forms a complex with the coordination number of two or more. In general, in the case of a transition metal, two- to nine-coordinated complexes are known in terms of the coordination number. The coordination number of 2 to 9 is preferred because technetium is a transition metal. The coordination number of 2 to 6 is more preferred in terms of electronic and steric stability of the complex. Further, it is known that technetium forms a six-, three-, or two-coordinated complex with a monodentate, bidentate, or tridentate ligand, which is stable *in vivo* (Non Patent Literatures 1 and 2). Thus, the coordination numbers of 2, 3 and 6 are still more preferred. For example, technetium forms a mixed ligand complex containing two-coordinated and three-coordinated complexes with D-Pen or AMPT at 1:2, and forms a six-coordinated complex with isonitrile.

The ^{99m}Tc complex that is the active ingredient of the ^{99m}Tc-labeled drug according to the present invention is composed of the ligand that is bound to a target molecule recognition element and forms a polycoordinated complex with Tc, and thus has the same number of target molecule recognition elements as that of ligands (FIG. 1). That is, the ^{99m}Tc complex has a plurality of binding sites to a target molecule. As described above, the complex composed of the ligand that is bound to a target molecule recognition element and forms a polycoordinated complex with Tc and consequently having the same number of target molecule recognition elements as that of ligands in the complex is referred to as polyvalent Tc complex, and a ^{99m}Tc-labeled drug comprising the polyvalent complex as an active ingredient is referred to as polyvalent Tc-labeled drug. For example, a ^{99m}Tc complex composed of a ligand that is bound to a target molecule recognition element and forms a two-coordinated complex with pentavalent Tc has two binding sites to a target molecule in the complex and is referred to as bivalent Tc complex. A ^{99m}Tc complex composed of a ligand that is bound to a target molecule recognition element and forms a three-coordinated complex with trivalent Tc has three binding sites to a target molecule in the complex and is referred to as trivalent Tc complex. A ^{99m}Tc complex composed of a ligand that is bound to a target molecule recognition element and forms a six-coordinated complex with monovalent Tc has six binding sites to a target molecule in the complex and is referred to as hexavalent Tc complex.

The complex that is the active ingredient of the ^{99m}Tc-labeled drug according to the present invention is a polyvalent complex, and exhibits a high affinity to a target molecule and high accumulation in the target molecule as compared to a monovalent complex.

Therefore, the polyvalent ^{99m}Tc-labeled drug according to the present invention exhibits high accumulation in a target site as compared to a conventionally used monovalent ^{99m}Tc-labeled drug, and thus, can be used without being further purified after its production. Further, the polyvalent ^{99m}Tc-labeled drug according to the present invention has sufficient *in vivo* stability.

As described above, the use of the polyvalent ^{99m}Tc-labeled drug according to the present invention can increase the accumulation of a radioactive complex in a target site, and consequently can provide high sensitivity in diagnostic imaging using the radiolabeled drug.

For example, a two-coordinated ^{99m}Tc complex that is composed of ^{99m}Tc and two molecules of D-Pen bound to a c(RGDfK) peptide having an affinity to integrin highly expressed in neovascular vessels in cancers is a bivalent complex having two binding sites to a target molecule integrin, and shows increased accumulation to a target site, neovascular vessels in cancers, as compared to the monovalent ^{99m}Tc complex. Therefore, the ^{99m}Tc-labeled drug comprising such complex can provide high sensitivity in the diagnosis of cancers.

In fact, the two-coordinated ^{99m}Tc complex that is composed of ^{99m}Tc and two molecules of D-Penbound to a c(RGDfK) peptide exhibited the same level of tumor accumulation even when not being purified after its production as a ^{99m}Tc complex that was produced using a tetradentate ligand and purified conventionally (see Example 2). Further, the bivalent ^{99m}Tc complex had sufficient *in vivo* stability (see Example 1). From those results, it is obvious that the ^{99m}Tc-labeled drug comprising such complex can provide high sensitivity in the diagnosis of cancers.

The ^{99m}Tc-labeled drug according to the present invention can be prepared, for example, by mixing a ligand bound to a target molecule recognition element with a ^{99m}Tc- containing pertechnetic acid or a salt thereof and a pertechnetate reducing agent. The pertechnetate reducing agent is used for reducing a pertechnetate salt into a low atomic valence state advantageous for forming a strong chelate compound. Various pharmaceutically acceptable pertechnetate reducing agents can be used as the pertechnetate reducing agent, and preferred examples thereof include a stannous salt and sodium dithionite. The stannous salt refers to a salt formed by bivalent tin, for example, the salt being formed with a halide ion such as a chloride ion or a fluoride ion, an inorganic acid residue ion such as a sulfate ion or a nitrate ion, or an organic acid residue ion such as a tartrate ion, an acetate ion, or a citrate ion.

Specifically, the ^{99m}Tc-labeld drug according to the present invention can be prepared by first adding a ^{99m}Tc solution to a lyophilized GH-kit to perform a reaction, to thereby prepare ^{99m}Tc-GH, and then mixing ^{99m}Tc-GH with a ligand bound to a target molecule recognition element. The kit contains 4 mg of α-D-glucoheptonic acid and 1.2 µg of stannous chloride in one vial and is adjusted so that the pH is 8 when pertechnetic acid (^{99m}TcO4-) is added.

Examples of the ligand bound to a target molecule recognition element include D-Pen bound to a c(RGDfK) peptide represented by the following formula (I).

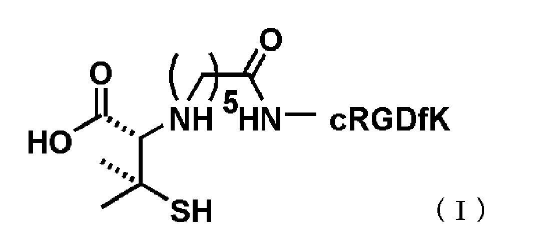

The D-Pen bound to a c(RGDfK) peptide represented by the above-mentioned formula (I) can be produced using a compound represented by the following formula (II).

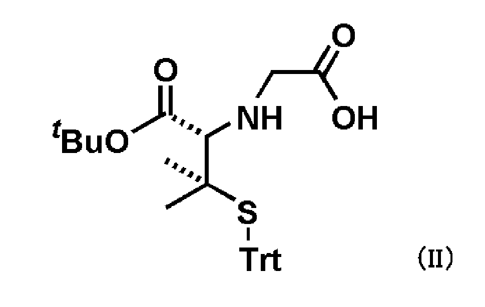

The ligand represented by the above-mentioned formula (I) is produced by crosslinking D-Pen and a c(RGDfK) peptide through hexanoic acid. In addition, preferred examples of the ligand bound to a target molecule recognition element include a ligand produced by crosslinking D-Pen and a c(RGDfK) peptide through polyethylene glycol.

One aspect of the radiolabeled drug according to the present invention can be a ¹⁸⁶Re- or ¹⁸⁸Re-labeled drug. The ¹⁸⁶Re- or ¹⁸⁸Re-labeled drug according to the present invention comprises a complex composed of a ligand that is bound to a target molecule recognition element and forms a polycoordinated complex with rhenium, and ¹⁸⁶Re or ¹⁸⁸Re. The ¹⁸⁶Re- or ¹⁸⁸Re-labeled drug according to the present invention can preferably be used as an internal radiation therapeutic drug because ¹⁸⁶Re and ¹⁸⁸Re both emit β rays with high energy exhibiting cytotoxicity.

Rhenium is a transition element belonging to Group VII like technetium, and has chemical nature similar to technetium. Thus, the ligand that forms a complex with technetium also forms a similar complex with rhenium.

Therefore, the above-mentioned ligand that forms a polycoordinated complex with technetium can be used as the ligand that forms the ¹⁸⁶Re complex or the ¹⁸⁸Re complex that is the active ingredient of the ¹⁸⁶Re- or ¹⁸⁸Re-lebeled drug. In actuality, non-radioactive ^{185 /187} Re that is a stable isotope of rhenium formed a two-coordinated complex with D-Pen. ^{185/187}Re also formed a two-coordinated complex with D-Pen bound to a c(RGDfK) peptide. The ligand is not limited to the ligands exemplified above, and any ligand can be used as long as the ligand is a compound capable of forming a polycoordinated complex with ¹⁸⁶Re or ¹⁸⁸Re.

The complex that is the active ingredient of the ¹⁸⁶Re- or ¹⁸⁸Re-labeled drug according to the present invention is a polyvalent complex, and exhibits an high affinity to a target molecule and high accumulation in the target molecule as compared to the monovalent complex.

Therefore, the polyvalent ¹⁸⁶Re- or ¹⁸⁸Re-labeled drug according to the present invention exhibits high accumulation in a target site, and thus can exert a high effect in internal radiation therapy. The polyvalent labeled drug according to the present invention has sufficient *in vivo* stability.

For example, a two-coordinated complex composed of ¹⁸⁶Re or ¹⁸⁸Re and D-Pen bound to a c(RGDfK) peptide having an affinity to integrin highly expressed in neovascular vessels in cancers is a bivalent complex having two binding sites to a target molecule integrin, and exhibits increased accumulation in a target site, neovascular vessels, in cancers as compared to the monovalent complex. Further, the bivalent ¹⁸⁶Re or ¹⁸⁸Re complex has sufficient in vino stability as is the case with the above-mentioned bivalent ^{99m}Tc complex. Therefore, the radiolabeled drug comprising the bivalent ¹⁸⁶Re or ¹⁸⁸Re complex as an active ingredient is highly useful as a therapeutic agent for cancer diseases.

The ¹⁸⁶Re- or ¹⁸⁸Re-labeled drug according to the present invention can be prepared, for example, by mixing a ligand bound to a target molecule recognition element with ¹⁸⁶Re or ¹⁸⁸Re-containing perrhenic acid or a salt thereof and a perrhenate reducing agent. The compounds exemplified above as the pertechnetate reducing agent can be used as the perrhenate reducing agent.

### Examples

Hereinafter, the present invention is more specifically described by way of examples. However, the present invention is by no means limited to the following examples.

### Example 1

A cyclic pentapeptide c(RGDfK) having an affinity to integrin highly expressed in neovascular vessels in cancer was used as a target molecule recognition element, and a ligand bound to this peptide was produced. In addition, a two-coordinated ^{99m}Tc complex was produced using this ligand. In addition, stability and pharmacokinetics of the produced ^{99m}Tc complex were evaluated. Further, the chemical structure of the complex produced by the complex formation of this ligand with ^{99m}Tc was examined using non-radioactive rhenium.

### (Experimental materials and methods)

### 1. General methods

A ⁹⁹Mo/⁹⁹mTc generator manufactured by FUJIFILM RI Pharma Co., Ltd. was used to produce a pertechnetic acid (^{99m}TcO4-). A silica gel plate (Silica gel 60F254 manufactured by Merck & CO., Inc.) was used for thin layer chromatography (TLC). Veronal buffer (pH 8.6) was used for cellulose acetate electrophoresis (CAE), and samples were electrophoresed at 1 mA for 30 minutes. For a reverse phase HPLC (RP-HPLC) column, a Cosmosil 5C18-AR 300 column (4.6 mm×150 mm, manufactured by Nacalai Tesque Inc.) was used, a mobile phase A (0.01 M phosphate buffer, pH 6.0) and a mobile phase B (MeOH) were employed, and a method comprising changing the ratio of the mobile phase B with a linear gradient of 0 to 18 minutes: B=0 to 60% and 18 to 21 minutes: B=60 to 100% (method 1) or a method comprising changing the ratio with a linear gradient of 0 to 18 minutes: B=0 to 60%,18 to 26 minutes: B=60 to 100%, and 26 to 31 minutes: B=100% (method 2) was used. A Cosmosil 5C18 AR 300 column (20 mm×150 mm, manufactured by Nacalai Tesque Inc.) was used for a preparative RP-HPLC column, and the sample was eluted at a flow rate of 5 mL/minute by a method comprising changing the ratio of the mobile phase B with a linear gradient of 0 to 5 minutes: B=0% and 5 to 45 minutes: B=0 to 100%. A JEOL-ALPHA 400 spectrometer (manufactured by JEOL Ltd.) was used for ¹H-NMR. FAB-MS was measured using a JEOL JMS-HX-110 A mass spectrometer (manufactured by JEOL Ltd.). MALDI-TO-MS was measured using a Kratos Axima CFR apparatus (manufactured by Shimadzu Corporation). Cyclo (Arg (Pbf) -Gly-Asp (O^{t}Bu) -D-Phe-Lys) was synthesized according to a method previously reported (Haubner R, Wester HJ, Reuning U, Senekowitsch-Schmidtke R, Diefenbach B, Kessler H, Stocklin G, and Schwaiger M., Radiolabeled alpha (v) beta 3 integrin in antagonists: a new class of tracers for tumor targeting. J. Nucl. med., 1999; 40: 1061-71). For any other reagent, a special grade reagent was used directly.

### 2. Synthesis of S-trityl-AMPT-N-acetate

S-Trityl-AMPT-N-acetate (S-Trt-AMPT-N-Ace) used as a ligand was synthesized. The synthesis is described below.

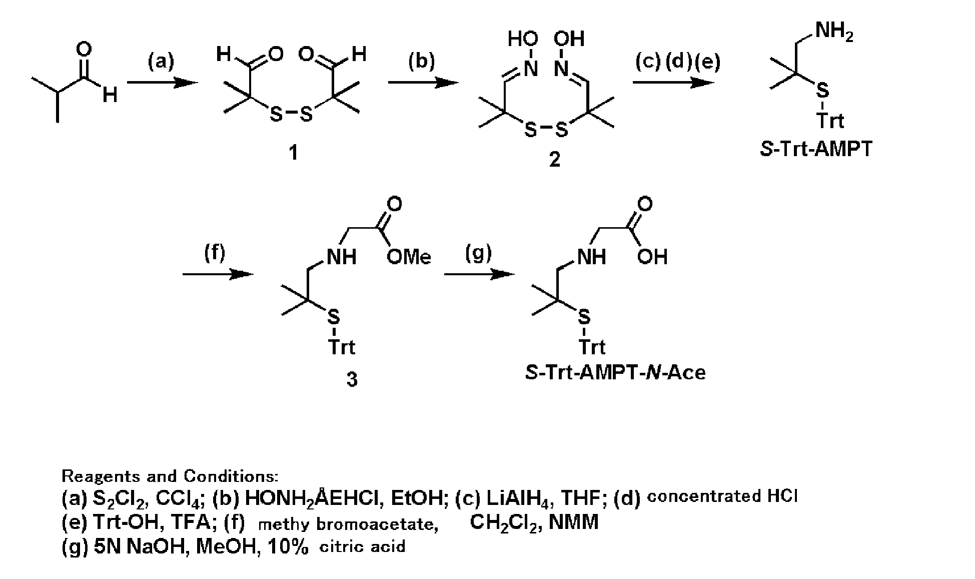

### (Experiment 1)

### Synthesis of 2,2,5,5-tetramethyl-3,4-dithiahexane-1,6-dial (1)

S₂Cl₂ (80 mL, 1.0 mol) was added dropwise to a solution of isobutylaldehyde (183mL, 2.0 mol) dissolved in dry CCl₄ (140 mL) while keeping a temperature of 50 to 55°C. HCl gas evolved in the reaction was removed with N₂ gas. The reaction solution was stirred for 2 hours, and then 10 N NaOH (110 mL) was slowly added dropwise to adjust pH to 10. Diethyl ether (100mL) was added, and an organic layer was washed with brine (100 mL) and then dried over MgSO₄. After the solvent had been distilled off, the residue was purified by distillation under reduced pressure (bp 113 to 114°C/6 mmHg) to yield a colorless oily substance, which was then cooled to yield Compound 1 (105.7 g, 51.2%) as a crystal. ¹H-NMR (CDCl₃) : δ 1.30 (s, 12H, CH₃), 9.00 (s, 2H, CHO); FABMS: m/z 207 [(M+H)⁺].

### (Experiment 2)

### Synthesisof2,2,5,5-tetramethyl-3,4-dithiahexane-1,6-dialbisoxime (2)

Compound 1 (4.12 g, 20 mmol) and hydroxylamine hydrochloride (4.17 g, 60 mmol) were dissolved in dry ethanol (20 mL). Then, 15 N NaOH (3.8 mL, 57 mmol) was added dropwise to that solution. The resulting solution was refluxed for 3 hours, and then water (100 mL) was added thereto. The solution was extracted with diethyl ether (4×20 mL), and the extract was dried over anhydrous Na₂SO₄. The solvent was distilled off to yield a crude product as an oily substance, which was then recrystallized from EtOAc-n-hexane to yield Compound 2 as a colorless crystal (2.48 g, 52.5%). ¹H-NMR (CDCl₃): δ 1.30 (s, 12H, CH₃), 7.30 (s, 2H, CH=NOH); FABMS: m/z 237 [(M+H)⁺].

### (Experiment 3)

### Synthesis of 1-amino-2-methyl-2-(S-trityl)-propanethiol (S-Trityl-AMPT, 3)

LiAlH₄ (482 mg, 12 mmol) dissolved in tetrahydrofuran (THF, 12 mL) was slowly added dropwise to Compound 2 (945 mg, 4 mmol) dissolved in THF (8 mL). The reaction solution was refluxed for 15 hours with vigorous stirring under a nitrogen flow, and then cooled to 0°C on ice. Ethyl acetate (3.2 mL) was added thereto, and then pH was adjusted to 1.0 by adding 6 N HCl. The solvent was distilled off, subsequently the residue was dissolved in trifluoroacetic acid (TFA, 11.4 mL), and then triphenylmethanol (2.1 g, 8 mmol) was added. After stirring under a nitrogen flow for 26 hours, TFA was distilled off under a nitrogen flow. The residue was dissolved in dichloromethane (DCM, 14 mL), and then, the pH of the solution was adjusted to 10 to 11 by the addition of 3 N NaOH. An organic layer was separated, and washed with water (3×7 mL), then saturated NaHCO₃ (2×3 mL), and brine (2×3 mL). The organic layer was dried over anhydrous Na₂SO₄, and then the solvent was distilled off. The residue was purified by silica gel chromatography with DCM-MeOH (10:1) as an eluent to yield Compound 3 as a pale brown oily substance (234 mg, 8.4%). ¹H-NMR (CDCl₃) : δ 1.00 (s, 6H, CH₃), 1.53 (s, 2H, NH₂), 1.76 (s, CH₂), 7.26-7.16 (m, 9H, aryl), 7.60-7.63 (d, 6H, aryl); FABMS: m/z 348 [(M+H)⁺].

### (Experiment 4)

### Synthesis of S-trityl-AMPT-N-acetate (S-Trt-AMPT-N-Ace)

Compound 3 (188 mg, 0.51 mmol) was dissolved in dry DCM (2 mL), and 5 N NaOH (0.1 mL) was added thereto. The reaction solution was stirred under a nitrogen flow for 1.5 hours, and then the solvent was distilled off. The residue was dissolved in water, and the pH of the solution was adjusted to 2.0 by the addition of 6 N HCl. The resulting precipitate was collected by filtration, and washed with 0.08 N HCl to yield S-Trt-AMPT-N-Ace as a pale gray crystal (36.2 mg, 72%). ¹H-NMR (CD₃OD): 1.20 (s, 6H, CH₃), 1.83 (s, 1H, NH), 3.09 (s, 2H, NHCH₂CO), 3.25 (s, 2H, SC(CH₃₎₂CH₂NH), 7.12-7.26 (m, 9H, aryl), 7.56-7.60 (d, 5H, aryl); FABMS: m/z 406 [(M+H)⁺].

### 3. Production of D-Pen(Trt)-O^{t}Bu-N-hexanoate (9)

D-Pen (Trt) -O^{t}Bu-N-hexanoate (9) used as a ligand was synthesized. The synthesis is described below.

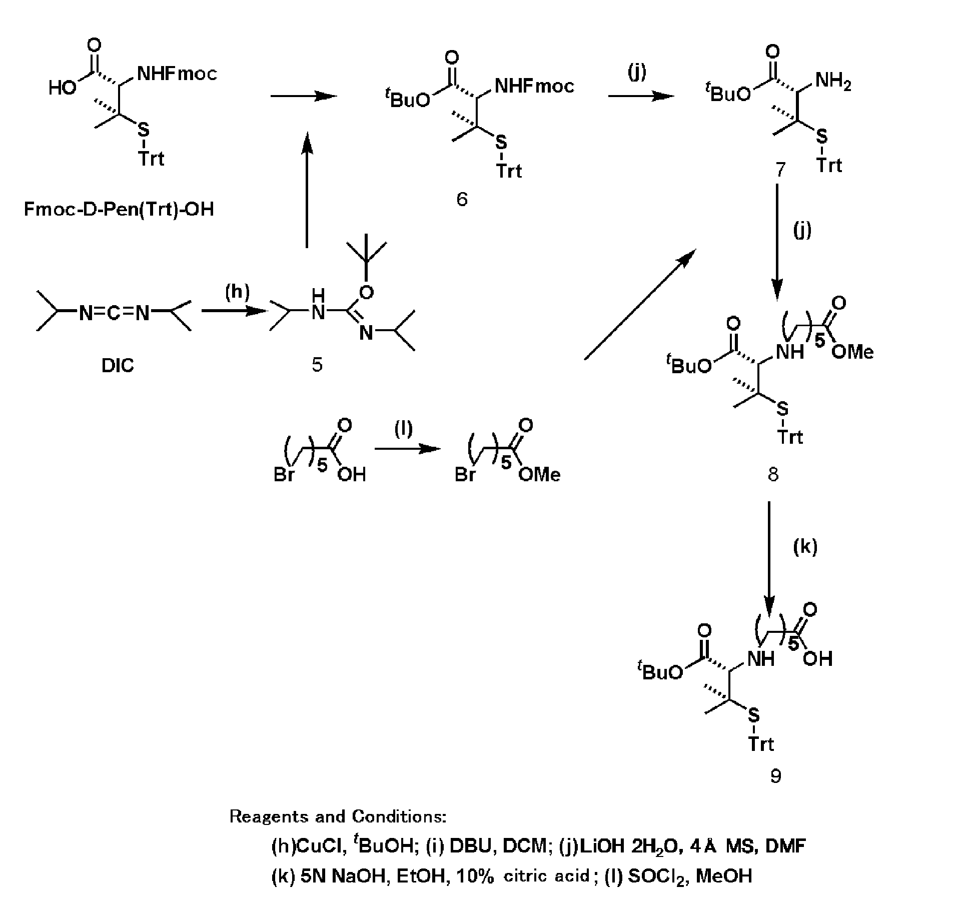

### (Experiment 5)

### Synthesis of N,N'-diisopropyl-O-tert-butylisourea (5)

tert-Butanol (2.78 g, 37.5 mmol) was added to a dry round-bottomed flask, and diisopropylcarbodiimide (DIC, 4.08 g, 32.3 mmol) and CuCl in a small amount were added thereto at 30°C under a nitrogen flow. The reaction solution was stirred at 30°C under a nitrogen flow for 4 days. Poly(4-Vinylpyridine) (cation exchange resin, 0.65 g) and DCM (16.5 mL) were added, and the resulting slurry solution was stirred for additional 15 minutes. A solid was filtrated off, and the eluted solution was distilled off to yield a crude crystal as a colorless clear oil (5.99 g, maximum 92.6%). This compound was used for the subsequent reaction without further purification. FABMS: m/z 201 [(M+H)⁺].

### (Experiment 6)

### Synthesis of Fmoc-D-penicillamine (Trt)-O^{t}Bu (6)

Fmoc-D-Penicillamine (Trt) -OH (500mg, 0.815mmol) was dissolved in DCM (15 mL), and Compound 5 (1.142 g, 5.703 mmol) was slowly added thereto. The mixture was stirred under a nitrogen flow for 15 hours, and then a precipitate was filtered off. The solvent in the filtrate was distilled off. Subsequently, the residue was purified by silica gel chromatography with n-hexane-Et₂O (2:1) as an eluent to yield Compound 6 as a white solid (520.9 mg, 95.4%) ¹H-NMR (CDCl₃) : δ 1.00 (s, 3H, CH₃), 1.11 (s, 3H, CH₃), 1.46 (s, 9H, tBu), 1.54 (brs, 1H, NH), 3.62-3.69 (d, 1H, COCHNH), 4.21-4.43 (m, 3H, CH₂CH-fluorenyl), 7.13-7.45 (m, 19H, aryl), 7.53-7.70 (d, 2H, aryl), 7.72-7.83 (d, 2H, aryl); FABMS: m/z 692 [(M+Na)⁺].

### (Experiment 7)

### Synthesis of D-penicillamine (Trt)-O^{t}Bu (7)

Fmoc-D-Penicillamine (Trt)-Ot^{B}u (500 mg, 0.764 mmol) was dissolved in dry DCM (40 mL), and then 1,8-diazabicyclo[5.4.0]-7-undecene (DBU) (1.14 mL, 7.46 mmol) was added thereto. The mixture was stirred at room temperature for 25 hours, and then the solvent was distilled off. The residue was purified by silica gel chromatography with n-hexane-EtOAc (3:1→2:1) as an eluent to yield Compound 7 as a colorless oily substance (280.9mg, 84.1%). ¹H-NMR (CDCl₃) : δ 1.08 (s, 3H, CH₃), 1.14 (s, 3H, CH₃), 1.28 (s, 9H, tBu), 7.10-7.26 (m, 9H, aryl), 7.55-7.61 (d, 2H, aryl); FABMS: m/z 448 [(M+H)⁺], 895 [(2M+H)⁺].

### (Experiment 8)

### Synthesis of methyl 6-bromohexanoate (4)

Methanol (25 mL) was cooled to -10°C, and SOCl₂ (2. 6 mL, 34 mmol) was slowly added dropwise thereto with stirring. After the dropwise addition, the mixture was stirred for 10 minutes, then 6-bromohexanoic acid (4.89 g, 25 mmol) was added, and the resulting mixture was stirred at room temperature overnight. The solvent was distilled off under reduced pressure. The residue was dissolved in ethyl acetate, and then the solution was washed with brine (3×30 mL). An organic layer was dried over anhydrous CaSO₄, and then the solvent was distilled off to yield Compound 4 as a colorless oily substance (5.13 g, 98%). ¹H-NMR (CD₃OD): δ 3.64 (s, 3H, OCH₃), (t, 2H, CH), 2.33 (t, 2H, CH₂α), (m, 2H, CH₂δ), (m, 2H, CH2β), (m, 2H, CH₂γ) ; FABMS: m/z 209 [(M+H)⁺].

### (Experiment 9)

### Synthesis of D-Pen(Trt)-O^{t}Bu-N-hexanoate methyl ester (8)

Activated 4Å molecular sieve (604.5 mg) was added to dry DMF (6 mL), and then LiOH·H₂O (96.03 mg, 2.289 mmol) was added thereto. This suspension was vigorously stirred for 10 minutes, then Compound 7 (476.5 mg, 1.06 mmol) dissolved in dry DMF was added thereto, and the resulting mixture was stirred for additional 30 minutes. Compound 4 (192.4 µL, 1.29 mmol) was added to this suspension, which was then stirred at room temperature for 24 hours. Inorganics were filtrated off, and then the residue was washed three times with DCM in a small amount. The filtrate was washed with water, and dried over anhydrous Na₂SO₄. The solvent was distilled off under reduced pressure, and subsequently the residue was purified by silica gel column chromatography under medium pressure with n-hexane-EtOAc (5:1 to 3:1) as an eluent to yield Compound 8 as a colorless clear oily substance (152.4 mg, 24.8%). ¹H NMR (CDCl₃) : δ 7.08-7.57 (m, 15H, Trt), 3.58 (s, 3H, OMe), 2.57 (s, 1H, COCHNH), 2.36-2.43 (m, 1H, -NH-), 2.22-2.26 (t, 2H, MeOCOCH₂), 1.331 (s, 9H, tBu), 1.248-1.667 (m, 6H, CH2 × 3), 0.94 (s, 3H, Me), 0.892 (s, 3H, Me); FABMS: m/z 576 [(M+H)⁺].

### (Experiment 10)

### Synthesis of D-Pen(Trt)-O^{t}Bu-N-hexanoate (9)

Compound 8 (326. 7mg, 0.567 mmol) was dissolved in a mixed solution of dry ethanol (40 mL) and 2.5 N NaOH (0.91 mL, 2.27 mmol). The mixture was subjected to a reaction at 40°C under a nitrogen flow for 3 hours, then DCM (15 mL) was added, and the resulting solution was washed with brine (15×2 mL). An organic layer was extracted, and the solvent was distilled off under reduced pressure. The residue was roughly purified by silica gel column chromatography with n-hexane-EtOAc (1:1) as an eluent to yield a crude product of Compound 9 as a colorless clear oily substance (282 mg, 88%). ¹H NMR (CDCl₃) : δ 7.60-7.62 (m, 15H, Trt), 2.52 (s, 1H, COCHNH), 2.28-2.31 (m, 3H, MeOCOCH₂ and NH), 1.38 (s, 9H, ^{t}Bu), 1.29-1.66 m, 6H, CH₂), 1.16 (s, 3H, Me), 1.02 (s, 3H, Me); FABMS: m/z 562 [(M+H)⁺], 584 [(M+Na)⁺].

### 4. Production of D-Pen-N-hexanoate-cRGDfK (11)

D-Pen-N-hexanoate-cRGDfK as the ligand bound to the cRGDfK peptide was produced using D-Pen (Trt) -O^{t}Bu-N-hexanoate (compound 9). The production is described below.

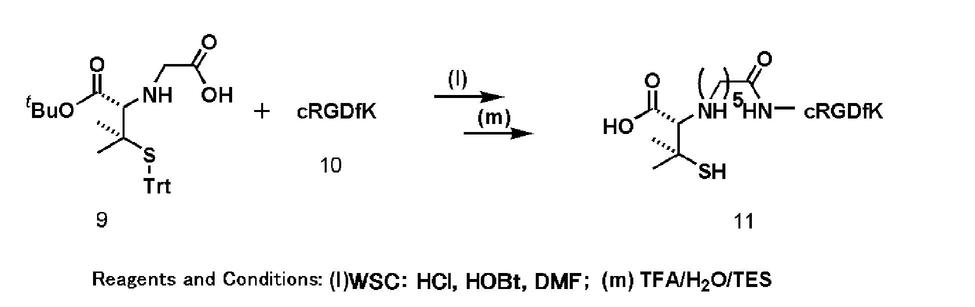

### (Experiment 11)

### Synthesis of D-Pen(Trt)-O^{t}Bu-N-hexanoate-cRGDfK peptide conjugate

WSC·HCl (89.19mg, 0.465 mmol) dissolved in dry DMF (15 mL) was slowly added to Compound 9 (23.6 mg, 0.423 mmol), Compound 10 (385.5 mg, 0.423 mmol), and hydroxybenzotriazole (HOBt, 71.23 mg, 0.465 mmol) dissolved in dry DMF (20 mL) on ice. The mixture was stirred at room temperature for 44 hours, and then the solvent was distilled off. The residue was dissolved in DCM (60 mL), and the resulting solution was washed with 5% citric acid (20 mL), water (20 mL), 5% NaHCO₃ (20 mL), and water (20 mL) sequentially, and finally washed with 5% citric acid (20mL). An organic layer was distilled off under reduced pressure, and then the residue was recrystallized from CH₃Cl-Et₂O under cooling with ice to yield a ligand bound to the protected amino acid as a pale gray crystal (545 mg, 88.6%). FABMS: m/z 1459 [(m+5H)⁵⁺].

### (Experiment 12)

### Synthesis of D-Pen-N-hexanoate-cRGDfK (11)

A mixed liquid of TFA, water, and triethylsilane (90:4.75:4.75, v/v/v, 81 mL) was added to the ligand bound to the protected amino acid (353.1 mg, 0.252 mmol), and the mixture was stirred at room temperature for 3 hours. The solvent was concentrated with nitrogen gas, and then the residue was dissolved in a minimum amount of water. Subsequently, a precipitate was produced by adding ethyl acetate. The precipitate was collected by filtration, and then washed with water and ethyl acetate. The precipitate was purified by preparative RP-HPLC to yield Compound 11 (35 mg, 30%) as a white solid. MALDI-TOF MS: m/z 849 [M], 850 [(M+H)⁺].

### 5. Production of [^{185/187}Re]-(D-Pen-N-acetate-c(RGDfK))₂ (12)

A two-coordinated ^{185/187}rhenium complex composed of ^{185/187}rhenium and a ligand bound to the cRGDfK peptide that is D-Pen-N-acetate-c(RGDfK) was produced. The production is described below.

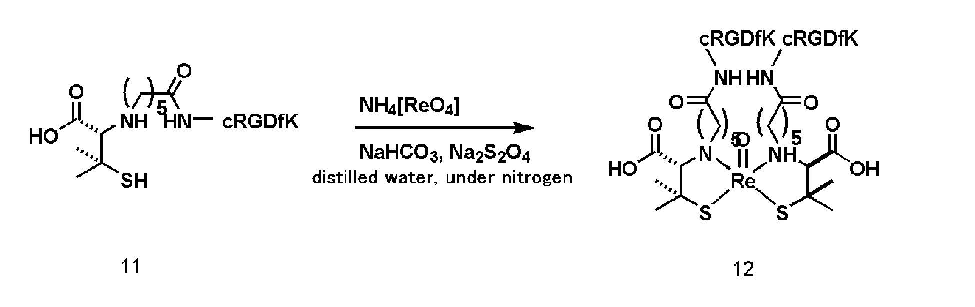

### (Experiment 13)

Ammonium perrhenate (3.38 mg, 12.6 µmol), Compound 11 (1 mg, 1.26 µmol), and 12.5 mM NaHCO₃ (0.2 mL, 2.52 µmol) were added to a reaction vessel and the vessel was sealed. After sending nitrogen gas to the reaction vessel for 15 minutes, a 0.25 M sodium dithionite solution (0.1 mL, 12.6 µmol) was added, and the mixture was stirred for 4 hours. Then, a product was analyzed by analytical RP-HPLC (method 2). MALDI-TOF MS: m/z 1784 [(M+2H)⁺].

### 6. Production of [^{185/187}Re]-(D-Pen)₂ (14)

A two-coordinated ^{185/187}rhenium complex composed of D-penicillamine and ^{185/187}rhenium was produced. The production is described below.

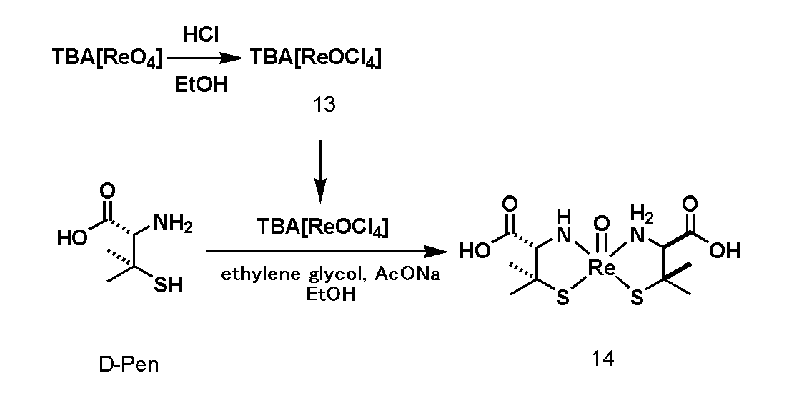

### (Experiment 14)

### Production of tetrabutyl ammonium [^{185/187}ReOCl₄] (13)

Tetrabutyl ammonium [ReO₄ (1.1 g, 2.2 mmol) was dissolved in ethanol (20 mL), and the reaction solution was saturated with hydrogen chloride gas under cooling with ice. The reaction solution was stirred at room temperature for 2 hours, and then concentrated under a nitrogen flow until the volume of the reaction solution was reduced by half. Subsequently, the resulting solution was left to stand in a freezer to yield a pale yellow crystal (0.5 g, 38%) FABMS: m/z 242 [M (TBA)], 243 [(M (TBA)+H)⁺], IR (KBr) : 2962, 2874, 1470, 1380, 1169, 737 cm⁻¹.

### (Experiment 15)

### Production of [^{185/187}Re]-(D-Pen)₂ (14)

TBA[ReOCl₄] (50 mg, 0.085 mmol) was dissolved in ethanol (6 mL), and ethylene glycol (0.43 mL) was added thereto with stirring. Sodium acetate was added thereto until the reaction solution exhibited a perse color. Subsequently, 1.2 mL of a solution of D-Pen (25.4 mg (0.171 mmol) /1.2 mL) in ethanol was added, and the mixture was stirred at room temperature for 2 hours. The pH of the reaction solution was adjusted to 9 by the addition of 1 N NaOH, and the solvent was distilled off. The residue was dissolved in a small amount of methanol, and analyzed (method 1) and purified by RP-HPLC. As the amount of the product was very small, its yield was not able to be calculated. ¹H-NMR (CD30D): δ 1.212 (s, 3H, Me β), 1.591 (s, 3H, Me β), 1.738 (s, 3H, Me β), 1.997 (s, 3H, Me β), 3.041 (s, 1H, CH α), 3.934 (s, 1H, CH α).

### 7. Production of ^{99m}Tc-glucoheptonate (^{99m}Tc-GH)

^{99m}Tc-GH was obtained by adding 1.0 mL of a ^{99m}Tc solution (370 MBq, 20 pmol) to a lyophilized GH kit including α-D-glucoheptonic acid (4.0 mg, 17.7 µmol) and 1.2 µg of SnCl₂. 2H₂O to perform a reaction at room temperature for 20 minutes. The production of ^{99m}Tc-GH was confirmed by TLC (acetone, Rf value=0, saline, Rf value=1.0).

### 8. Production of ^{99m}Tc-(D-penicillamine)₂

D-penicillamine (D-Pen, 1.19 mg, 8 µmol) was dissolved in 400 µL of 0.1 M Tris hydrochloride buffer (pH 9.0) purged with nitrogen. Subsequently, this solution was sequentially diluted with 0.1 M Tris hydrochloride buffer (pH 9.0) to prepare solutions of 2 mM, 0.2 mM, 0.02 mM, 0.01 mM, and 0.002 mM ligand. 100 µL of the ^{99m}Tc-GH solution was added to 100 µL of the solution at each ligand concentration, and after mixing, the mixture was subjected to a reaction at room temperature for 1 hour. The production of ^{99m}Tc-(D-Pen)₂ was confirmed by RP-HPLC (method 1) and CAE.

### 9. Production of ^{99m}Tc-(AMPT-N-acetate)₂

TFA (200 µL, 2.6 mmol) was added to S-Trt-AMPT-N-Ace (3.24 mg, 8 umol), and the mixture was stirred for 1 minute. TES (10 µL, 74 µmol) was added to this solution. After confirming that the color of the reaction solution was changed from yellow to colorless, the solvent was distilled off with nitrogen. 0.1 M Tris hydrochloride buffer (pH 9.0) (400 µL) purged with nitrogen was added thereto. Subsequently, this solution was sequentially diluted with 0.1 M Tris hydrochloride buffer (pH 9.0) to prepare solutions of 2 mM, 0.2 mM, 0.02 mM, 0.01 mM, and 0.002 mM ligand. 100 µL of a ^{99m}Tc-GH solution ([GH]=4.0 mg/mL) was added to 100 µL of the solution at each ligand concentration, the mixture was sufficiently mixed, and then subjected to a reaction at room temperature for 1 hour. The production of the product was confirmed by RP-HPLC (method 1) and CAE.

### 10. Production of ^{99m}Tc-(D-Pen-N-Hx-cRGDfK)₂

D-Pen-N-hexanoate-cRGDfK (Compound 11, 0.8 mg, 1 µmol) was dissolved in 50.5 µL of 0.1 M Tris hydrochloride buffer (pH 9.0) purged with nitrogen. Subsequently, this solution was diluted with 0.1 M Tris hydrochloride buffer (pH 9. 0) to prepare a solution of 2 mM ligand. 100 µL of a ^{99m}Tc-GH solution was added to 100 µL of the ligand solution, and after sufficiently mixing, the mixture was subjected to a reaction at room temperature for 1 hour. The production of the compound was confirmed by RP-HPLC (method 2).

### 11. Examination of stability

Each ^{99m}Tc-labeled compound was purified by RP-HPLC (method 1 or method 2), and then the solvent was distilled off under reduced pressure. The residue was redissolved in 400 µL of 0.01 M phosphate buffer (pH 6.0). This solution was stored at room temperature, and after 1, 3, 6, and 24 hours, its radiochemical purity was analyzed by CAE or RP-HPLC.

### 12. Animal experiment

100 µL (about 1 µCi) of unpurified ^{99m}Tc-(D-Pen-N-hexanoate-cRGDfK)₂ or ^{99m}Tc-(D-Pen-N-hexanoate-cRGDfK)₂purifiedbyRP-HPLCwas administered to the tail veins of ddY-strain male mice at 6 weeks of age (three mice for each group) . The mice were sacrificed 10 minutes, 1, 3, and 6 hours after the administration, and blood samples were collected. The mice were anatomized, and the weight and radioactivity of each organ were measured. The radioactivity in urine and feces excreted by 6 hours after the administration was also measured.

### (Results)

### 1 . Effects of ligand concentration on production of technetium complex

^{99m}Tc- (D-Pen) with a radiochemical yield of 95% or more was obtained when the concentration of D-Pen was 0.01 mM or more. However, the radiochemical yield was found to reduce when the concentration of D-Pen was less than 0.01 mM (FIG. 2). Meanwhile, ^{99m}TC- (AMPT-N-acetate) ₂ with a radiochemical yield of 90% or more was obtained when the concentration of AMPT-N-acetate was 0.01 mM or more, but the radiochemical yield was found to abruptly reduce when the concentration was less than 0.01 mM (FIG. 2).

### 2. Stability of ^{99m}Tc complex in buffer

The stability of each of ^{99m}Tc-(D-Pen)₂ and ^{99m}Tc- (AMPT-N-acetate) ₂ in a buffer was examined, and ^{99m}Tc- (D-Pen) ₂ showed higher stability (FIG. 3).

Thus, D-Pen was selected as the ligand, and a ligand derivative into which c(RGDfK) had been introduced was produced and was labeled with ^{99m}Tc. The stability of the resulting ^{99m}Tc-(D-Pen-N-Hx-cRGDfK)₂ in the buffer was examined. ^{99m}Tc- (D-Pen-N-Hx-cRGDfK)₂ was present as an unchanged form even after 6 hours (FIG. 4) . That is, it was proved that ^{99m}Tc- (D-Pen-N-Hx-cRGDfK) ₂ was stable in the buffer.

Further, Re-(D-Pen-Hx-cRGDfK)₂ was produced using non-radioactive rhenium (Re) in order to identify the structure of ^{99m}Tc-(D-Pen-N-Hx-cRGDfK)₂. It was found by mass spectrometry that Re and the ligand derivative formed a complex at 1:2. The retention time of ^{99m}Tc-(D-Pen-N-Hx-cRGDfK)₂ and the retention time of Re- (D-Pen-Hx-cRGDfK) ₂ in RP-HPLC were identical to each other (Table 1) .

**[Table 1]**

| Compound | Retention time (minutes) |
|---|---|
| [^{99m}Tc] - (D-pen)₂ | 14.1 |
| [^{185/187}Re] - (D-Pen)₂ | 11.4 |
| [^{99m}Tc] - (D-Pen-hx-cRGDfK)₂ | 21.3 |
| [^{185/187}Re] - (D-Pen-hx-cRGDfK)₂ | 20.6 |

From the results, ^{99m}Tc- (D-Pen-Hx-cRGDfK) ₂ is thought to be a complex including ^{99m}Tc and the ligand derivative at 1:2. As described above, the drug design of the present invention has been found to be useful for the development of a new ^{99m}Tc-radioactive pharmaceutical having an enhanced affinity to a target.

### 3. Pharmacokinetics of ^{99m}Tc-(D-Pen-Hx-cRGDfK)₂

^{99m}Tc-(D-Pen-Hx-cRGDfK)₂ was administered to mice. Accumulation of both purified one and unpurified one in the stomach was not observed (FIGS. 5A and 5B, and FIGS. 6A and 6B). When ^{99m}Tc is dissociated from the complex *in vivo,* ^{99m}Tc is accumulated in the stomach. Thus, it is indicated that the complex of the present invention was stable *in vivo.* Those results indicate that the bivalent ^{99m}Tc complex that is stable *in vivo* is obtained by using D-Pen as the ligand and binding the target molecule recognition element thereto. Those results also strongly suggest that the polyvalent ^{99m}Tc-labeled drug that is sufficiently stable *in vivo* is obtained by appropriately selecting the ligand.

### Example 2

Using nude mice in which tumor was transplanted, the accumulation of ^{99m}Tc- (D-Pen-Hx-cRGDfK)₂ in the tumor was compared with that of ^{99m}Tc-TMEC-[N-hexanoate-c(Arg-Gly-Asp-D-Phe-Lys)]₂ (hereinafter, sometimes abbreviated as ^{99m}Tc-TMEC-RGD₂) produced from a conventional tetradentate ligand. The accumulation was examined using an unpurified sample of synthesized ^{99m}Tc-(D-Pen-Hx-cRGDfK)₂, and an unpurified sample and a purified sample of synthesized ^{99m}Tc-TMEC-RGD₂ .

### (Experimental materials and methods)

### 1. Tumor model

Human glioma cell line, U87MG cells at 5×10⁶ cells/mouse were administered subcutaneously in the left femur of Balb/c-nu/nu mice at 4 weeks of age. The mice bearing the tumor of 0.5 g in size were used for a body distribution experiment.

### 2. Production of ^{99m}Tc-(D-Pen-Hx-cRGDfK)₂

A ^{99m}Tc-GH solution with [GH]=4.0 mg/mL was obtained by adding 750 µL (1.58 µCi) of a solution of Na[^{99m}TcO₄] in saline eluted from a ⁹⁹Mo/^{99m}Tc generator to a lyophilized GH kit (3.0 mg) including α-D-glucoheptonic acid and stannous chloride to perform a reaction at room temperature for 20 minutes. Further, a 2 mM ligand solution was separately prepared by dissolving D-Pen-Hx-cRGDfK (0.8 mg, 0.942 µmol) in 471 µL of 0.1 M Tris hydrochloride buffer (pH 9.0) purged with nitrogen. 140 µL of the ^{99m}Tc-GH solution was added to 140 µL of the ligand solution, they were sufficientlymixed, and then subj ected to a reaction at 40°C for 40 minutes. The reaction solution was diluted to 40-fold with saline so that [Free ligand]=0.025 mM to use as the unpurified sample to be administered.

### 3. Production of ^{99m}Tc-TMEC-RGD₂

A ^{99m}Tc-GH solution with [GH]=4.0 mg/mL was obtained by adding 500 µL (970 µCi) of a solution of Na[^{99m}TcO₄] in saline eluted from a ⁹⁹Mo/^{99m}Tc generator to a lyophilized GH kit (2.0 mg) including α-D-glucoheptonic acid and stannous chloride to perform a reaction at room temperature for 20 minutes. Further, a 2 mM ligand solution was separately prepared by dissolving TMEC-[N-hexanoate-c(Arg-Gly-Asp-D-Phe-Lys)]₂ (hereinafter, sometimes abbreviated as TMEC-RGD₂) (0.7 mg, 0.406 µmol) in 200 µL of 0.1 M acetate buffer (pH 3.5) purged with nitrogen. 200 µL of the ^{99m}Tc-GH solution was added to 200 µL of the ligand solution, they were sufficiently mixed, and then subjected to a reaction at 80°C for one hour. A half amount of the reaction solution (200 µL) was applied to RP-HPLC (mobile phase: A=10 mM phosphate buffer (pH 6.0), B=MeOH; gradient system: 0 to 18 minutes: B=0 to 60%, 18 to 26 minutes: B=60 to 100%, 26 to 31 minutes: B=100%) , a fraction eluted at 24 minutes was collected, the organic solvent was distilled off under reduced pressure, and then the residue was diluted with 10% ethanol/saline to use as the purified sample. Further, the other half amount of the reaction solution (200 µL) was diluted to 40-fold with saline so that [Free ligand]=0.025 mM to use as the unpurified sample to be administered.

TMEC-RGD₂ was synthesized as described below using D-Pen (Trt) -O^{t}Bu-N-hexanoate (9) synthesized by the method described in Example 1.

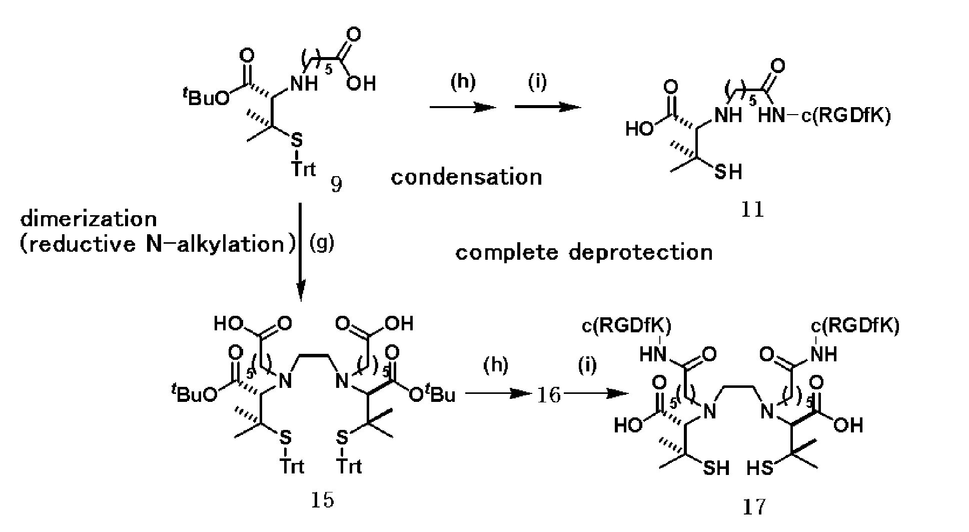

### (Experiment 1)

### Synthesis of TMEC(Trt)₂-O^{t}Bu-N-hexanoate (15)

Compound 9 (50 mg, 0.089 mmol) dissolved in MeOH (9 mL) was cooled with ice, glyoxal (40wt% in H₂O, 6. 6 µL, 0.062 mmol) was added followed by addition of NaBH₃CN (5.6mg, 0.089 mmol) under a nitrogen atmosphere, and the mixture was stirred at 40°C. Glyoxal (40wt% in H₂O, 6.6 µL, 0.062 mmol) and NaBH₃CN (5.6 mg, 0.089 mmol) were added every other day, and the mixture was stirred for 3 days. Water (5 mL) and 5% NaHCO₃ (5 mL) were gradually added to the reaction solution to decompose NaBH₃CN, and then MeOH was distilled off under reduced pressure. The residue was diluted with CHC₃ (10 mL), and the solution was washed with 5% NaHCO₃ (10 mL) and brine (10 mL). Then, a CHCl₃ layer was washed with 5% citric acid (10 mL), and subsequently washed with water (10 mL) and brine (10 mL). An organic layer was concentrated under reduced pressure, and the residue was purified by silica gel column chromatography with n-hexane-EtOAc (3:1) as an eluent to yield Compound 15 as a pale yellow oily substance (24 mg, 47%) . ¹H-NMR (CDCl₃) : δ 0.91 (s, 6H, Me × 2), 1.23-1.26 (overlapped, 4H, CH₂ × 2), 1.29 (s, 6H, Me _{×} 2) , 1.38-1.41 (m, 4H, CH₂ × 2), 1.46 (s, 18H, ^{t}Bu × 2) , 1.54-1.62 (m, 4H, CH₂ × 2), 2.25 (s, 2H, αCH), 2.28-2.32 (overlapped, 8H, CH₂ × 4), 2.52 (brs, 2H, NHCH₂), 2.70 (brs, 2H, NHCH₂), 7.13-7.57 (m, 30H, aromatic (Trt)); FABMS: m/z 614 [(M+2K)²⁺/2], Found 614. MALDI-TOF MS (cynapinic acid as a matrics) : 614 [(M+2K)²⁺/2] , Found 614. ESIMS: m/z 614 [(M+2K)²⁺/2] . Found 614.

### (Experiment 2)

### Synthesis of TMEC(Trt)₂-O^{t}Bu-[N-hexanoate-c(Arg(Pbf)-Gly-Asp(O^{t}Bu)-D-Phe-Lys]₂ (16)

Compound 15 (50 mg, 0.044 mmol), ECDI (83.4 mg, 0.091 mmol), and HOBt (14.7 mg, 0.096 mmol) were dissolved in DMF (1.5 mL), and a solution of WSCl (18.4 mg, 0.096 mmol) in DMF (1.5 mL) was gradually added dropwise thereto with stirring under cooling with ice. The mixture was stirred at room temperature for 48 hours, and then the solvent was distilled off under reduced pressure. The residue was dissolved in CH₂Cl₂ (7 mL), and the resulting solution was washed with (a) 5% citric acid (7 mL), brine (3 mL), (b) water (7 mL), brine (7 mL), (c) 10% NaHCO₃ (8 mL), brine (5 mL), (d) water (10 mL), brine (5 mL), and (e) 5% citric acid (10 mL), brine (5 mL), sequentially. An organic layer was dried over anhydrous sodium sulfate, and then the solvent was concentrated under reduced pressure. The residue was dissolved in a minimum amount of CHCl₃, and then Et₂O in a small amount was added to precipitate a product. The precipitate was collected by filtration, and washed with Et₂O in a small amount to give Compound 16 as a white gray solid (106 mg, 82.1%), which was used directly for the subsequent reaction without further purification.

### (Experiment 3)

### Synthesis of TMEC-[N-hexanoate-c(Arg-Gly-Asp-D-Phe-Lys)]₂ (17)

Compound 16 (106 mg, 0.036 mmol) was dissolved in TFA/water/triethylsilane (90:4.75:4.75 (v/v/v), 11.7 mL), and the mixture was stirred at room temperature for 6 hours. The reaction solution was concentrated under a nitrogen flow, and Et₂O (2 mL) was gradually added to the resulting residue to precipitate a product. The precipitate was washed three times by further addition of Et₂O (2 mL) and decantation. Finally, the precipitate was washed by centrifuging an Et₂O suspension of the precipitate (1000 rpm, 5 minutes×2) to yield a crude product of Compound 17 as a gray solid. The resulting precipitate was purified by preparative RP-HPLC (system 3) and lyophilized to yield Compound 17 as a white solid (15.4 mg, 25%) . MALDI-TOFMS: m/z 1723 [(M+H)⁺] , 862 [(M+2H)²⁺/2], Found 1723, 862.

### 4. Examination of accumulation of radiolabeled drug in tumor and organ

100 µL of the unpurified ^{99m}Tc- (D-Pen-Hx-cRGDfK) ₂ sample (about 1.1 µCi) was administered to the tail veins of tumor-bearing Balb/c-nu/nu male mice at 10 weeks of age. There were 5 mice in one group. The mice were sacrificed by beheading 1 hour after the administration. Blood, organs of interest, and the tumor were removed, and the weight of and the radioactivity in each organ were measured.

100 µL of the purified or unpurified ^{99m}Tc-TMEC-RGD₂ sample (about 0.5 µCi) was administered to the tail veins of tumor-bearing Balb/c-nu/nu male mice at 10 weeks of age. There were 4 mice in one group. The mice were sacrificed by beheading 1 hour after the administration. Blood, organs of interest, and the tumor were removed, and the weight of and the radioactivity in each organ were measured.

### (Results)

The accumulation of ^{99m}Tc-TMEC-RGD₂ produced from the conventional tetradentate ligand was low when using the unpurified form that contains the excessive ligand. Its accumulation was increased by removing the excessive ligand by purification (Table 2). This result confirmed that the excessive ligand inhibited the accumulation of ^{99m}Tc-TMEC-RGD₂ in the tumor.

Meanwhile, the accumulation of unpurified ^{99m}TC-(D-Pen-Hx-cRGDfK)₂ that contains the excessive ligand was the same level as that of ^{99m}Tc-TMEC-RGD₂ from which the excessive ligand was removed (upper rows in Table 2) . Further, the ratio of unpurified ^{99m}Tc- (D-Pen-Hx-cRGDfK)₂ accumulated in the tumor to that in each tissue was equivalence to or higher than the ratio of purified ^{99m}Tc-TMEC-RGD₂ accumulated in the tumor to that in each tissue (lower rows in Table 2). It should be noted that, in Table 2, ^{99m}Tc- (Pen-RGD) ₂ means ^{99m}Tc- (D-Pen-Hx-cRGDfK)₂.

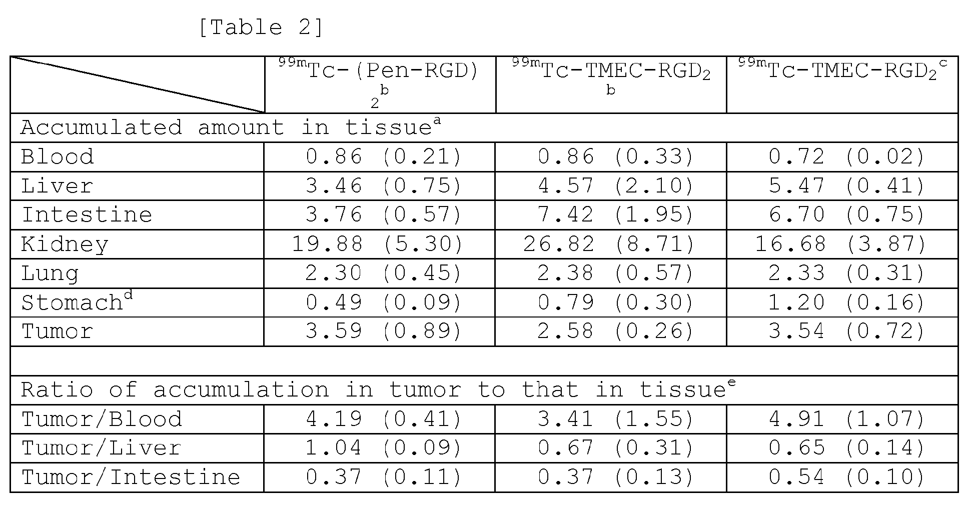

### Symbols in Table 2 are described below.

a: Represented by ratio of radioactivity per 1 g of each tissue to administered radioactivity (% injected dose/gram tissue).
b: Administered without removing the excessive ligand.
c: Administered after removing the excessive ligand by HPLC.
d: Represented by ratio to administered radioactivity (% injected dose)
e: Calculated from % injected dose/gram tissue in the tumor and the tissue.

As apparent from the above, ^{99m}TC-(D-Pen-Hx-cRGDfK)₂ as the bivalent ^{99m}Tc-labeled drug produced by the complex formation the monovalent ligand with ^{99m}Tc is efficiently accumulated in the target site even when the complex is unpurified, and thus, unpurified ^{99m}Tc- (D-Pen-Hx-cRGDfK)₂ can be used directly for diagnostic imaging and the like.

### Example 3

Pen-PEG₃-RGD as a D-penicillamine (D-Pen) derivative was synthesized based on the novel drug design in which the bivalent ^{99m}Tc-lebeled drug was produced by the complex formation of the monovalent ligand with ^{99m}Tc. Penicillamine and c(RGDfK) were crosslinked with hexanoic acid in D-Pen-N-hexanoate-cRGDfK (11) synthesized in Example 1, while penicillamine and c(RGDfK) were crosslinked with ethylene glycol in Pen-PEG₃-RGD.

Pen-PEG₃-RGD was synthesized as follows. First, a mercapto group of D-Pen was protected with a trityl group and a carboxyl group thereof was protected with ^{tert}Bu. Ethyl ester in a compound obtained by a reaction of the protected D-Pen with ethyl 11-bromo-3, 6, 9-oxaundecanate synthesized separately was hydrolyzed, and then the compound was bound to the cyclic RGD peptide c(RGDfK). Then, the resulting compound was deprotected to yield the objective compound.

### Industrial Applicability

The radiolabeled drug according to the present invention greatly contributes to the fields of the elucidation of pathological conditions, diagnostic imaging, and the development of pharmaceuticals.

## Claims

1. A radiolabeled drug showing increased accumulation in a target site, comprising a complex composed of a ligand that is bound to a compound capable of binding to a target molecule and forms a polycoordinated complex with a metal, and a radionuclide of the metal.

2. A ^{99m}Tc-labeled drug for diagnosis showing increased accumulation in a target site, comprising a complex composed of D-penicillamine or 1-amino-2-methylpropane-2-thiol-N-acetate, which is a ligand that is bound to a compound capable of binding to a target molecule and forms a two-coordinated or three-coordinated complex with pentavalent technetium (Tc), and ^{99m}Tc.

3. A ^{99m}Tc-labeled drug for diagnosis according to claim 2, wherein the ligand that is bound to a compound capable of binding to a target molecule is a ligand that is bound to a cyclic pentapeptide c(RGDfK) .

4. A¹⁸⁶Re- or ¹⁸⁸Re-labeled drug for treatment showing increased accumulation in a target site, comprising a complex composed of D-penicillamine or 1-amino-2-methylpropane-2-thiol-N-acetate, which is a ligand that is bound to a compound capable of binding to a target molecule and forms a two-coordinated or three-coordinated complex with pentavalent rhenium (Re), and ¹⁸⁶Re or ¹⁸⁸Re_{.}

5. A ¹⁸⁶Re- or ¹⁸⁸Re-labeled drug for treatment according to claim 4, wherein the ligand that is bound to a compound capable of binding to a target molecule is a ligand that is bound to a cyclic pentapeptide c(RGDfK).

6. A radiolabeled drug according to any one of claims 1 to 5, the radiolabeled drug being used in diagnosis or treatment.

7. A ligand for preparing a radiolabeled drug for diagnosis or treatment showing increased accumulation in a target site, the ligand being bound to a compound capable of binding to a target molecule and forming a polycoordinated complex with a metal.

8. A ligand for preparing a radiolabeled drug according to claim 7, wherein a radionuclide of the metal comprises any one of radionuclides of metals selected from the group consisting of ^{99m}technetium, ¹⁸⁶rhenium_{,} and ¹⁸⁸rhenium.

9. A ligand for preparing a radiolabeled drug according to claim 8, wherein the ligand that forms a polycoordinated complex with a metal is D-penicillamine or 1-amino-2-methylpropane-2-thiol-N-acetate, which forms a two-coordinated or three-coordinated complex with pentavalent technetium or rhenium.

10. A ligand for preparing a radiolabeled drug according to claim 8 or 9, wherein the ligand that is bound to a compound capable of binding to a target molecule is a ligand that is bound to a cyclic pentapeptide c(RGDfK).

11. A kit, comprising, as separate package units, a drug that comprises the ligand for preparing a radiolabeled drug of any one of claims 7 to 11, and a drug that comprises a radionuclide of a metal that forms a polycoordinated complex with the ligand.

12. A method of producing a compound represented by the following formula (I): 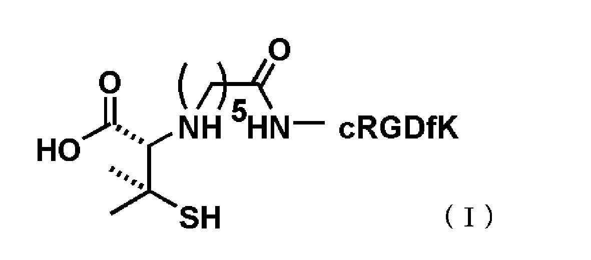 the method comprising using a compound represented by the following formula (II):

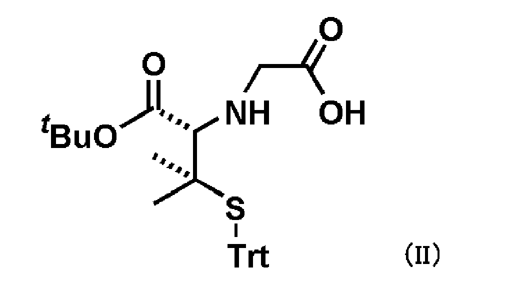

13. A method of increasing accumulation of a radiolabeled drug in a target site, the method comprising using a radiolabeled drug that comprises a complex composed of a ligand that is bound to a compound capable of binding to a target molecule and forms a polycoordinated complex with a metal, and a radionuclide of the metal.

14. A method according to claim 13, wherein the radionuclide of the metal is any one of radionuclides of metals selected from the group consisting of ^{99m}technetium, ¹⁸⁶rhenium, and ¹⁸⁸rhenium.

15. A method according to claim 14, wherein the ligand that forms a polycoordinated complex with a metal is D-penicillamine or 1-amino-2-methylpropane-2-thiol-N-acetate, which forms a two-coordinated or three-coordinated complex with pentavalent technetium or rhenium.

16. A method according to claim 14 or 15, wherein the ligand that is bound to a compound capable of binding to a target molecule is a ligand that is bound to a cyclic pentapeptide c(RGDfK).
